# EUROPEAN PATENT APPLICATION

(11) **EP 4 443 443 A1**
(43) Date of publication of application: **09.10.2024**
(21) Application number: 24168045.3
(22) Date of filing: 02.04.2024
(51) Int. Cl.: G16H 80/00, G16H 40/20, G16H 10/60, G16H 15/00

(54) **PROCESS AND SYSTEM FOR CONNECTING A PATIENT AND A SPECIALIST BASED ON A PATHOLOGY**

(30) Priority: 05.04.2023 IT 202300006666
(71) Applicant: Tartaglione, Palma, 27199 Pavia (IT); Gionti, Vincenzo, 27100 Pavia (IT)
(72) Inventor: Tartaglione, Palma, 27199 Pavia (IT); Gionti, Vincenzo, 27100 Pavia (IT)
(74) Representative: Garavelli, Paolo

(57) **Abstract**

A system and a process are described for connecting a patient and a specialist based on a pathology, via a medical system configured as at least one terminal device (1) connected to a data acquisition server (SAD); the process comprises the steps of: acquiring and sending to the data acquisition server (SAD) a name or a series of information about the patient pathology (INFO.PATH) and a filter for a selected geographical area (ID.GEO); acquiring and sending to the terminal device (1) a name of a known pathology (PATH) that has a set of keywords in common with the name or set of information of the patient pathology (INFO.PATH); confirming, if relevant, the name of a known pathology acquired through a source (S.PATH.CERT) or ignoring the suggestion and proceeding with the insertion of free text that refers to the name of a known pathology (ID.PATH).

## Description

The present invention concerns a process and a system for connecting a patient and a specialist based on a pathology.

In general, the present invention relates to Query processing by sorted data search, Query formulation in natural language or dialogue systems, ICT specially adapted for the management or administration of healthcare resources or facilities; ICT specially adapted for the management or operation of medical equipment or devices for the management or administration of healthcare resources or facilities, e.g. healthcare workforce management, ICT specially adapted for therapies or health improvement plans, e.g. for managing prescriptions, for guiding therapy or for monitoring patient compliance, ICT specially adapted for the management or operation of medical equipment or devices for the management or administration of healthcare resources or facilities, e.g. healthcare personnel management, ICT specially adapted for the management or processing of patient-related medical or healthcare data for patient-specific data, e.g. for electronic health records, ICT specially adapted for the management or administration of healthcare resources or facilities; ICT specially adapted for the management or operation of medical equipment or devices for the operation of medical equipment or devices for remote operation.

In particular, the present invention relates to Query processing using string matching techniques, specially adapted for medical diagnosis, medical simulation or medical data mining; ICT specially adapted to detect, monitor or model epidemics or pandemics for computer-aided diagnosis, e.g. based on expert medical systems, ICT specially adapted for the management or processing of patient-related medical or healthcare data for patient-specific data, e.g. for electronic medical records, ICT specially adapted to facilitate communication between specialists or patients, e.g. for collaborative diagnosis, therapy or health monitoring.

The state of the art is represented by patent CN 104965898 B regarding an online query system to optimize hospital resources and allow the patient to identify the department they belong to and the doctors relevant to that department.

The system solves the problem of identifying the professional corresponding to a professional specialty by limiting the search within a single hospital. Furthermore, the list of pathologies and specialists is linked to a physical database. The query system does not go into the merits of rare diseases and is not able to analyse aspects of unrecognized diseases and appropriately identify the specialist's areas of expertise.

Patent application US 2018/0068075 A1 is known, and deals with a system that allows the association between a list of doctors and registered patients.

Doctors must be registered and are invited to enter their area of specialty and the pathologies treated.

The system of association of doctors with patients does not use any algorithm for searching the list of specialists, for example, an algorithm based on expertise with respect to a specific pathological condition. Therefore, there is no list of specialists based on any requests for visits and, consequently, there is no database containing a list of specialists constantly updated and enriched with specialists for new pathologies.

Furthermore:
- there is a lack of control over the areas of expertise entered independently by specialists;
- a score is not defined for each specialist calculated on the basis of the degree of patient satisfaction, the availability and the extent of the specialist's scientific production;
- there is no database of patients registered or invited to enter their information;
- there is no search engine in which the pathology can be entered by filtering by geographical extension;
- there is no process for requesting an outpatient or remote visit via a tele-visit;
- in case of unavailability of the specialist, it is not possible to propose a valid alternative in order to act promptly.

Patent application WO 2021/139477 A1 is known and deals with the implementation of a process in which the probability of illness is calculated based on the symptoms and on the basis of the illness by associating the most appropriate department in order to avoid incorrect bookings which lead to an increase in waiting lists and lengthening of times in the diagnosis and treatment of the pathology.

The search criterion is based on symptoms rather than pathology and, therefore, the identification of the most up-to-date specialist is carried out in a macro-area of the specialty, for example, chest pain is associated with the Cardiology department, with the risk of not finding the specialist closest to the pathology. Furthermore, the search for a specialist carried out by macro-area involves a rough correlation with the result of excluding the correlation by areas of expertise, for example, one can find a general cardiologist, but not a cardiologist specialized in electrophysiology.

In particular, the state of the art is represented by the following patent documents: US 2022/297135 A1, WO 2017/093608 A1, WO 02/080043 A1, and CN 113 058 729 A.

Object of the present invention is providing a process and a system for connecting a patient and a specialist based on a pathology of interest so that the result has a high degree of convergence with the solution sought, with the aim of ensuring to the patient with a correct preventive, diagnostic and therapeutic path.

A further object is providing a process and a system for connecting a patient and a specialist based on a pathology to allow the patient to acquire an updated list in real time of the most competent specialists based on the pathology, specialists possibly filtered by area geographical based on the pathology of interest.

A further object is providing a process and a system for connecting a patient and a specialist on the basis of a pathology following an indication of a pathology and possibly a geographical area in which to limit the search, selecting the specialist from the proposed list of specialists and sending a request to visit the specialist via the system manager.

The above and other objects and advantages of the invention, as will appear from the following description, are achieved with a process and a system for connecting a patient and a specialist based on a pathology, such as that described in the respective independent claims. Preferred embodiments and non-trivial variants of the present invention form the subject of the dependent claims.

It is understood that all the attached claims form an integral part of this description.

It will be immediately obvious that countless variations and modifications can be made to what is described (for example relating to shape, dimensions, arrangements and parts with equivalent functionality) without departing from the scope of the invention as appears from the attached claims.

The present invention will be better described by some preferred embodiments, provided by way of example and not by way of limitation, with reference to the attached drawings, in which:

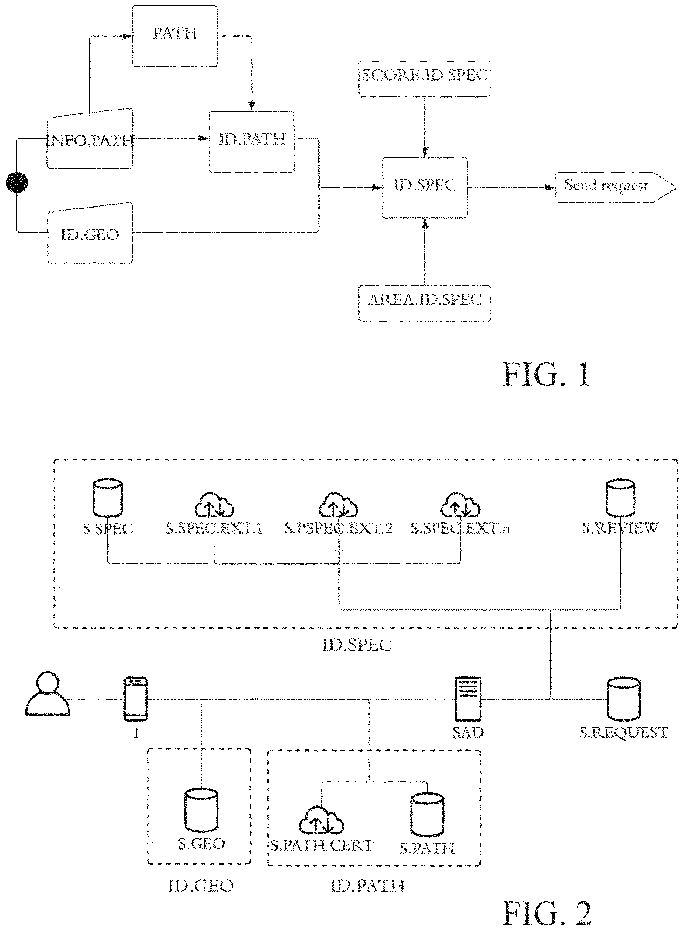

- FIG. 1 shows a flowchart of an embodiment of the process for connecting a patient and a specialist based on a pathology according to the present invention;
- FIG. 2 shows a functional diagram of an embodiment of the system for connecting a patient and a specialist based on a pathology according to the present invention; and

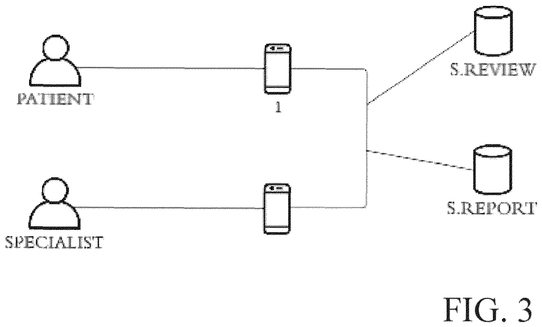

- FIG. 3 shows a further functional diagram following the diagram in the previous figure.

Referring to the Figures, it is possible to note that the process according to the present invention for connecting a patient and a specialist based on a pathology is carried out via a medical system configured as at least one terminal device 1 connected to a SAD data acquisition server.

Advantageously, this process comprises the following steps:
- via a terminal device 1, acquiring and sending to the SAD data acquisition server a name or series of information on the patient's pathology INFO.PATH and, if selected, a geographical area in which to limit the search ID.GEO;
- through at least one certified external source S.PATH.CERT and/or through an updated internal source S.PATH, acquiring and sending to the terminal device 1 a name of a known pathology PATH that has a series of keywords in common with the name or the series of information of the patient's pathology INFO.PATH, in order to allow the patient to receive a name of a known pathology ID.PATH to better identify the pathology of interest;
- using terminal device 1, confirming, if relevant, the name of a known pathology or ignoring the suggestion and proceeding with the insertion of free text that refers to the name of a known pathology ID.PATH;

- through an updated internal source S.GEO, acquiring, if selected, a geographical area in which to limit the ID.GEO search;
- via a further external source of a plurality of further external sources S.SPEC.EXT.1, S.SPEC.EXT.2, ..., S.SPEC.EXT.n and a further internal source of a plurality of further internal sources S.SPEC, acquiring and processing a list of names of ID.SPEC specialists that are automatically linked to the name of a known pathology ID.PATH, the list of names of ID.SPEC specialists is updated in real time and equipped with an updated score SCORE.ID.SPEC;
- using terminal device 1, choosing the name of at least one specialist from the list of ID.SPEC specialist names;
- via the terminal device 1, sending a request for a specialist visit to the chosen specialist to the SAD data acquisition server;
- via the SAD data acquisition server, in addition to the patient's data, saving the name of a known pathology ID.PATH, upon acceptance by the patient via a digital tick of sensitive data processing according to current legislation;
- via a further internal source of a plurality of further internal S.SPEC sources, inserting or updating the data of the chosen specialist in the list of names of ID.SPEC specialists; and
- defining a connection between the patient and the specialist chosen from the list of names of ID.SPEC specialists, to allow opening a health record of a plurality of health records collected in an internal S.REPORT source.

It is possible to acquire, with the help of an artificial intelligence algorithm, a list of unregistered specialists coming from the plurality of additional external sources, S.SPEC.EXT.1, S.SPEC.EXT.2, ..., S.SPEC .EXT.n, retrieved in real time by invoking a series of APIs, application programming interfaces, i.e. a set of processes that act as an intermediate level that processes the transfer of data between systems through a documented interface, of external sources, for example NCBI, National Center for Biotechnology Information, based on the extent of scientific production, i.e. on the quantity, type and publication date of the specialist's scientific production on the topic of interest.

This series of external source APIs is invoked in order to acquire a list of publications relating to the researched topic and for each of them, by invoking further APIs, obtain a record, which in turn can be broken down into smaller units, called fields (e.g. author, title of the article, title of the periodical, date of publication, MESH terms) from which it is possible to extract the essential information for identifying the specialist.

It is possible to acquire a list of already registered specialists from the plurality of additional internal S.SPEC sources by searching a constantly updated internal database containing the description of the fields of interest AREA.ID.SPEC, for example, precision oncology, cryo-balloon ablation, pituitary craniopharyngioma, and the specialist's score. This list of specialists has the advantage of making each specialist easily available as they are already registered on the portal.

The fields of interest AREA.ID.SPEC are acquired in two ways:
- entered directly by the specialist;
- through an analysis of the scientific production of the specialist obtained through API invocation of external sources, for example NCBI, National Center for Biotechnology Information, from which the description of potential fields of interest can emerge useful for defining the area of expertise in detail of the specialist author of the publication.

The list of ID.SPEC specialist names is updated in real time and given a SCORE.ID.SPEC score, based on:
- the extent of scientific production on the topic, i.e. the quantity, type and date of publication of scientific production on the topic;
- the availability of specialists already registered in the portal, translated based on the response time to previous consultation requests and compared to a history of the activity of the specialist chosen from the list of names of ID.SPEC specialists; and
- a score relating to the degree of patient satisfaction S.REVIEW associated with the specialist chosen from the list of names of ID.SPEC specialists.

The process also comprises the steps of:
- in an internal S.REQUEST source, storing information relating to the visit request forwarded by the patient, i.e. patient's data, name of the pathology sought, data of the specialist chosen from the list of names of ID.SPEC specialists and, if indicated, geographical area in which to limit the ID.GEO search;
- in an internal S.PATH source, storing the series of information on the pathology indicated by the patient, in the form of comments, symptoms, diagnoses; and
- in an internal S.SPEC source, updating / inserting the data of the specialist chosen in the list of names of ID.SPEC specialists in order to open a health record of a plurality of health records collected in an internal S.REPORT source.

Each intermediate process in the set of API intermediate processes is performed by an artificial intelligence algorithm.

The process allows retrieving information relating to biomedical scientific literature by invoking a series of APIs from at least one external source, comprising NCBI, National Center for Biotechnology Information.

A computer program comprises computer program code means adapted to perform all or part of the steps of the process according to the present invention when such program is executed on a computer.

This computer program is contained on a computer-readable medium.

A medical system according to the present invention for connecting a patient and a specialist based on a pathology, is configured as at least one terminal device 1 connected to a SAD data acquisition server, and is arranged to allow carrying out the steps of a process for connect a patient and a specialist based on a pathology.

Advantageously, such a medical system comprises:
- at least one certifie3d external source S.PATH.CERT and/or an updated internal source S.PATH capable of acquiring a name of a known pathology PATH that has a series of keywords in common with the name or a series of information of the pathology of the patient INFO.PATH, in order to allow the patient to receive a name of a known pathology ID.PATH to better identify the pathology of interest;
- a plurality of additional external sources S.SPEC.EXT.1, S.SPEC.EXT.2, ..., S.SPEC.EXT.n and a plurality of additional internal sources S.SPEC, designed to acquire and process a list of names of ID.SPEC specialists who are automatically linked to the name of a known pathology ID.PATH;

- a plurality of additional internal sources S.SPEC, designed to insert the data of the chosen specialist into the list of names of ID.SPEC specialists; and
- an internal source S.REPORT designed to collect and allow the opening of a plurality of health files.

Furthermore, the medical system comprises:
- an internal source S.REQUEST, designed to store the information relating to the visit request forwarded by the patient, i.e. patient's data, name of the pathology sought, data of the specialist chosen from the list of names of ID.SPEC specialists and, if indicated, geographical area ID.GEO in which to limit the search;
- an internal source S.PATH, designed to store the series of information on the pathology indicated by the patient, in the form of comments, symptoms, diagnoses; and
- an internal source S.SPEC, designed to update/enter the data of the specialist chosen in the list of names of specialists ID.SPEC and containing the description of the fields of interest of the specialists AREA.ID.SPEC.

A processing device of a medical system comprises a processor capable of acquiring and processing a list of names of specialists ID.SPEC linked to the name of a known pathology ID.PATH, with respect to:
- a further external source of a plurality of further external sources S.SPEC.EXT.1, S.SPEC.EXT.2, ..., S.SPEC.EXT.n; and
- a further internal source of a plurality of further internal sources S.SPEC.

The object of the present invention concerns a process for putting the patient in contact with the most appropriate specialist based on the pathology.

The patient searches for a specialist by indicating a pathology and possibly a geographical area in which to limit the search, selecting the specialist from the list of specialists proposed and sending a request to visit the specialist via the system manager.

The process concerns a first step of research and selection of a specialist and a second step of requesting and planning a visit.

The process comprises the steps of:
- indicating a pathology; and eventually
- indicating a geographical area relating to the specialist;
- selecting a specialist;
- requesting and scheduling a specialist visit.

The process is based on an initial association of the words used to describe the pathology with the words from a database used to describe a plurality of known pathologies.

The first association of the words used to describe the pathology with the words in the database allows the search for the known pathology to converge with the pathology searched for.

The database that allows the description of a plurality of known pathologies is based on the list defined by the international system of classification of diseases, traumas, surgical interventions and diagnostic and therapeutic processes, ICD-9-CM International Classification of Diseases, 9th revision - Clinical Modification.

This database updated in real time can be accessed via Internet.

The next step of the process is to identify a list of specialists for the identified pathology.

The list of specialists, generated with the help of an artificial intelligence algorithm, comes from two sources consulted in parallel:
a. list of specialists registered in a constantly updated internal database containing the description of their respective fields of interest, for example, precision oncology, cryo-balloon ablation, pituitary craniopharyngioma, and the specialist's score.
   The fields of interest are indicated in two ways:
   - directly from the specialist;
   - through an analysis of the text of the specialist's scientific production obtained from the National Center for Biotechnology Information, NCBI, the description of potential fields of interest can emerge useful for defining in detail the area of expertise of the specialist who authored the publication.
      This list of specialists has the advantage of making each specialist easily available as they are already registered in the constantly updated portal.
b. list of unregistered specialists retrieved in real time by invoking a series of APIs, application programming interfaces, from external sources, for example NCBI, National Center for Biotechnology Information, based on the extent of scientific production, i.e. on the quantity, type and date of publication of the specialist's scientific production on the topic of interest. This step may comprise, by way of example, the following steps:
   - invoking a series of external source APIs that return a list of publications relating to the searched topic;
   - for each publication obtained, a further API is in turn invoked which returns a record, which in turn can be broken down into smaller units, called fields (e.g.: author, article title, periodical title, date of publication, MESH terms) and the essential information for identifying the specialist is therefore extracted.

The list of specialists thus obtained is further filtered taking into consideration the extent of scientific production on the topic.

The choice of specialist is based on an updated score, based on:
- the extent of scientific production on the topic, i.e. the quantity, type and date of publication of scientific production on the topic;
- the availability of specialists already registered in the portal, translated on the basis of a response time to previous consultation requests and compared to a history of the activity of the specialist chosen from the list of names of specialists;
- a score relating to the degree of patient satisfaction associated with the specialist chosen from the list of names of specialists.

In summary, the main stages of the process are:
- Step 1: in which the patient makes a request for a visit with a specialist and waits for a response. It consists of the following steps:
   - inserting keyword (pathology) and possibly filter by geographical area;
   - searching for a specialist;
   - selecting the specialist;
   - sending a visit request.
- Step 2: in which the patient has the possibility to choose the slot (day and time) of the visit/television (defined based on the availability of the specialist.

Summarizing, the present invention refers to a process for connecting a patient and a specialist based on a pathology, via a medical system configured as at least one terminal device (1) connected to a data acquisition server (SAD), the process comprising the steps of:
- via the terminal device (1), acquiring and sending to the data acquisition server (SAD) a name or series of information of the patient's pathology (INFO.PATH) and a filter for a selected geographical area (ID.GEO);
- through at least one source (S.PATH.CERT), (S.PATH), acquiring and sending to the terminal device (1) a name of a known pathology (PATH) that has a series of keywords in common with the name or the series of information relating to the patient pathology (INFO.PATH), in order to allow the patient to receive a name for a known pathology (ID.PATH) to better identify the pathology of interest;
- using the terminal device (1), confirming, if relevant, the name of a known pathology acquired via a source (S.PATH.CERT) or ignore the suggestion and proceeding with the insertion of free text that refers to the name of a known pathology (ID.PATH).

The invention also deals with a medical system to connect a patient and a specialist based on a pathology, configured as at least one terminal device (1) connected to a data acquisition server (SAD), designed to allow carrying out the steps of the above process to connect a patient and a specialist on the basis of a pathology. The medical system comprises:
- at least one external source (S.PATH.CERT), (S.PATH) capable of acquiring a name of a known pathology (PATH) that has a series of keywords in common with a name or a series of information of the pathology of the patient (INFO.PATH), in order to allow the patient to receive a name for a known pathology (ID.PATH) to better identify the pathology of interest;
- an updated source (S.GEO) designed to acquire a filter for a selected geographical area (ID.GEO);
- a plurality of further sources (S.SPEC.EXT.1, S.SPEC.EXT.2, ..., S.SPEC.EXT.n), (S.SPEC), designed to acquire and process a list of names of specialists (ID.SPEC) who are automatically linked to the name of a known pathology (ID.PATH); and
- a source (S.REPORT) designed to collect and allow the opening of a plurality of health files.

The above medical system further comprises:
- a source (S.REQUEST), designed to store the information relating to the visit request forwarded by the patient, i.e. patient data, name of a sought pathology, data of the specialist chosen from the list of names of specialists (ID.SPEC) and a filter for a selected geographical area (ID.GEO);
- a source (S.PATH), designed to store the series of information on the pathology indicated by the patient, in the form of comments, symptoms, diagnoses; and
- at least one source (S.SPEC), suitable for updating/inserting the data of the specialist chosen in the list of names of specialists (ID.SPEC) and containing the description of the fields of interest of the specialists (AREA.ID. SPEC).

Finally, the invention deals with a processing device of the above medical system, the processing device comprising a processor capable of acquiring and processing a list of names of specialists (ID.SPEC) linked to the name of a known pathology (ID.PATH), as compared to:
- a further source of a plurality of further sources (S.SPEC.EXT.1, S.SPEC.EXT.2, ..., S.SPEC.EXT.n), (S.SPEC).

## Claims

1. Process for connecting a patient and a specialist based on a pathology, via a medical system configured as at least one terminal device (1) connected to a data acquisition server (SAD), **characterized in that** it comprises the steps of:
- via the terminal device (1), acquiring and sending to the data acquisition server (SAD) a name or series of information of the patient's pathology (INFO.PATH) and a filter for a selected geographical area (ID.GEO);
- through at least one source (S.PATH.CERT), (S.PATH), acquiring and sending to the terminal device (1) a name of a known pathology (PATH) that has a series of keywords in common with the name or the series of information relating to the patient pathology (INFO.PATH), in order to allow the patient to receive a name for a known pathology (ID.PATH) to better identify the pathology of interest;
- using the terminal device (1), confirming, if relevant, the name of a known pathology acquired via a source (S.PATH.CERT) or ignore the suggestion and proceeding with the insertion of free text that refers to the name of a known pathology (ID.PATH).

2. Medical system to connect a patient and a specialist based on a pathology, configured as at least one terminal device (1) connected to a data acquisition server (SAD), designed to allow carrying out the steps of a process to connect a patient and a specialist on the basis of a pathology according to the previous claim, **characterized in that** the medical system comprises:
- at least one external source (S.PATH.CERT), (S.PATH) capable of acquiring a name of a known pathology (PATH) that has a series of keywords in common with a name or a series of information of the pathology of the patient (INFO.PATH), in order to allow the patient to receive a name for a known pathology (ID.PATH) to better identify the pathology of interest;
- an updated source (S.GEO) designed to acquire a filter for a selected geographical area (ID.GEO);
- a plurality of further sources (S.SPEC.EXT.1, S.SPEC.EXT.2, ..., S.SPEC.EXT.n), (S.SPEC), designed to acquire and process a list of names of specialists (ID.SPEC) who are automatically linked to the name of a known pathology (ID.PATH); and
- a source (S.REPORT) designed to collect and allow the opening of a plurality of health files.

3. Medical system according to the previous claim, **characterized in that** it comprises:
- a source (S.REQUEST), designed to store the information relating to the visit request forwarded by the patient, i.e. patient data, name of a sought pathology, data of the specialist chosen from the list of names of specialists (ID.SPEC) and a filter for a selected geographical area (ID.GEO);
- a source (S.PATH), designed to store the series of information on the pathology indicated by the patient, in the form of comments, symptoms, diagnoses; and
- at least one source (S.SPEC), suitable for updating/inserting the data of the specialist chosen in the list of names of specialists (ID.SPEC) and containing the description of the fields of interest of the specialists (AREA.ID.SPEC).

4. Processing device of a medical system according to claim 3 or 4, **characterized in that** it comprises a processor capable of acquiring and processing a list of names of specialists (ID.SPEC) linked to the name of a known pathology (ID.PATH), as compared to:
- a further source of a plurality of further sources (S.SPEC.EXT.1, S.SPEC.EXT.2, ..., S.SPEC.EXT.n), (S.SPEC).
